# FASCICULE DE BREVET EUROPEEN

(11) **EP 1 035 826 B1**
(45) Date de publication et mention de la délivrance du brevet: **09.10.2002**
(21) Numéro de dépôt: 98958290.3
(22) Date de dépôt: 01.12.1998
(51) Int. Cl.: A61K 7/035, A61K 7/48, A61K 7/06

(54) **PRODUIT D'HYGIENE CAPILLAIRE ET/OU CORPORELLE POUR LES HOMMES ET LES ANIMAUX**
HAAR- UND/ODER KÖRPERPFLEGEMITTEL FÜR MENSCHEN UND TIERE
HAIR AND/OR BODY HYGIENIC PRODUCT FOR HUMAN BEINGS AND ANIMALS

(30) Priorité: 02.12.1997 FR 9715173
(43) Date de publication de la demande: 20.09.2000
(73) Titulaire: Benoit, Jean-Pierre, 14800 Deauville (FR); Bac, Elisabeth, 14800 Deauville (FR)
(72) Inventeur: Benoit, Jean-Pierre, 14800 Deauville (FR); Bac, Elisabeth, 14800 Deauville (FR)
(74) Mandataire: Nargolwalla, Cyra
(86) Numéro de dépôt international: FR9802581
(87) Numéro de publication internationale: WO99027899

(56) Documents cités:
- WO-A-92/09260
- DE-A- 2 752 320
- DE-A- 4 443 644
- FR-A- 2 555 441
- US-A- 4 330 438
- BASE DE DONN ES "CHEMICAL ABSTRACTS" (SERVEUR: STN), Abr.117:97 087, Colombus, OH, USA; & JP 04 049 224 A (KAO K.K.) 18 F VRIER 1992 XP002074250

## Description

L'invention a pour objet un produit d'hygiène capillaire et/ou corporelle pour les hommes et les animaux, qui se présente sous la forme d'une poudre applicable directement sur la chevelure ou sur le corps.

Elle a également pour objet le procédé de fabrication de ce produit.

Le produit selon la présente invention peut être utilisé en tant que shampooing pour laver la chevelure et/ou le corps, comme produit démêlant pour les cheveux, en tant que produit "après-shampooing", ou encore comme un produit exfoliant pour le corps.

Plusieurs formes de shampooing sont connues dans l'art antérieur. La forme classique reste le shampooing liquide. Néanmoins, il existe également des shampooings sous forme de gels, de crèmes, ou de mousses aérosol.

Des shampooings sous forme solide ont également déjà été proposés dans l'art antérieur. Mais il s'avère que ces shampooings "solides" se présentent toujours sous la forme de produits mis en forme, par exemple de barres, de galets ou de comprimés. On peut ainsi se référer aux brevets ou demandes de brevets suivants : US 4 012 341, WO93/07245, EP 0 330 435.

C'est ainsi également que l'on peut citer la demande de brevet FR 95 08265, déposée par les inventeurs, qui décrit un produit d'hygiène capillaire pour l'homme ou de traitement pileux pour l'animal, ce produit se présentant sous forme de comprimés caractérisés par un temps de délitement assez court sur les cheveux. Ce produit se révèle très facile à transporter et stable à la conservation, même en atmosphère humide. Cependant, cette présentation sous forme de comprimé entraine certains inconvénients. Il a notamment été constaté à l'usage que les comprimés ne se délitent pas de façon totalement satisfaisante dans le cas des chevelures abondantes. Ces problèmes semblent provenir essentiellement du fait que les cheveux doivent être très abondamment mouillés pour que le comprimé donne des résultats acceptables.

Mais en tout état de cause, il n'a jamais été sérieusement envisagé dans l'art antérieur de pouvoir appliquer directement sur la chevelure ou sur le corps un shampooing sous forme d'une poudre, car on ne pouvait pas imaginer qu'une telle application pourrait conduire à des résultats satisfaisants.

Bien entendu, on peut relever que des shampooings sous forme de poudre ont déjà été proposés, mais il convient de souligner qu'il s'agissait toujours de shampooings à dissoudre avant l'emploi, ce qui, au niveau de l'utilisation, conduit au même schéma que celui d'un shampooing liquide.

Charles Zviak, dans "Science des traitements capillaires" (Masson, 1988) a ainsi décrit des shampooings sous forme de poudre à dissoudre au moment de l'emploi, afin d'obtenir un shampooing liquide. Il est indiqué dans cet ouvrage que les shampooings sous forme de poudre à dissoudre au moment de l'emploi sont de moins en moins utilisés, bien qu'ils constituent la forme la moins chère. Selon Zviak, ceci provient du fait qu'ils ne sont pas très pratiques à l'utilisation. A cela s'ajoute la difficulté d'ajouter des ingrédients de qualité à un produit pulvérulent dont on attend qu'il se dissolve très facilement au moment de l'emploi.

Le brevet DE 44 43 644 décrit une préparation solide coulante obtenue par granulation en lit fluidisé de pâtes tensioactives et non une poudre. Par ailleurs, bien que ces préparations contiennent une proportion de tensioactifs de 40 % au maximum, elles ne contiennent pas une proportion importante de parfum.

A la connaissance des inventeurs, le seul document ayant mentionné l'utilisation d'un shampooing sous forme de poudre est la demande de brevet DE 4214480. Ce document décrit, plus que sommairement, un procédé pour l'utilisation d'un shampooing capillaire réduit en poudre, ce procédé étant caractérisé par le fait que l'on saupoudre sur les cheveux humides un shampooing réduit en poudre par dessiccation, lequel se lie à l'eau pour donner un shampooing normal.

Mais, à la lecture des six lignes que comporte cette demande de brevet, il s'avère qu'il s'agit là d'une formulation classique de shampooing liquide qui a été séchée pour la mettre sous forme de poudre.

Du fait de ce procédé de séchage du shampooing liquide, la poudre résultante ne contient bien évidemment que de la matière active, c'est-à-dire essentiellement des agents tensio-actifs.

Etant donné cette teneur extrêmement élevée en matières actives (en fait proche de 100%), l'inventeur de la demande de brevet allemand précitée pouvait effectivement espérer obtenir un résultat acceptable en appliquant le shampooing liquide déshydraté directement sur les cheveux. Mais il convient de relever qu'aucune précision sur la composition et qu'aucune qualification des résultats obtenus n'apparaissent dans la description, on ne peut plus succincte, de cette demande de brevet.

Toujours est-il que la poudre en question, obtenue donc par dessiccation de shampooing liquide, ne peut en aucun cas se révéler économiquement viable, ni apporter les avantages qui vont être décrits ci-après en relation avec le produit conforme à l'invention.

Celui-ci est caractérisé par le fait qu'il se présente sous forme de poudre ne contenant tout au plus que 40% de matières actives, c'est-à-dire de produits lavants ou de produits moussants.

De ce fait, le produit sous forme de poudre selon la présente invention offre la possibilité de comprendre d'autres produits tels que parfums, colorants, anti-oxydants, vitamines, huiles essentielles, matières végétales, algues marines, et ce en quantités notablement plus élevées que dans un shampooing liquide normal ou dans la poudre obtenue par séchage d'un shampooing liquide telle que décrite dans la demande de brevet DE 4214480.

De façon surprenante et inattendue, le produit d'hygiène capillaire et/ou corporelle sous forme de poudre selon la présente invention possède un pouvoir lavant et un pouvoir moussant très satisfaisants. Et ceci en dépit du fait qu'il s'agit d'une poudre contenant un pourcentage de matières actives bien inférieur au seul shampooing sous forme de poudre mentionné dans l'art antérieur.

Le produit d'hygiène capillaire et/ou corporelle pour les hommes et les animaux objet de la présente invention, est ainsi caractérisé par le fait qu'il se présente sous la forme d'une poudre applicable directement sur la chevelure et/ou sur le corps et qu'il comprend moins de 40%, de préférence moins de 35%, et plus préférentiellement encore moins de 30%, d'au moins un agent tensio-actif, ces pourcentages étant exprimés en poids par rapport au poids total de la poudre. Le complément à 100% est constitué par un ou plusieurs produits choisis dans le groupe comprenant les sucres, les amidons, les celluloses, les polyols, les protéines, les acides aminés, les parfums, les colorants, les anti-oxydants, les matières végétales, les algues marines, les vitamines, les huiles essentielles et les charges minérales.

Les agents tensio-actifs utilisés dans le produit d'hygiène capillaire et/ou corporelle conforme à l'invention peuvent être des tensio-actifs de type anionique, cationique ou polymère cationique, non ionique ou amphotère.

Les tensio-actifs anioniques sont préférés, du fait de leur bon pouvoir lavant et moussant. On peut citer à titre d'exemples les alkyl sulfates, tels que les lauryl sulfates, les alkyl et aryl sulfonates, les oléfinesulfonates et les alkyl sulfonates secondaires, les alkyl éther sulfates et les esters de l'acide sulfosuccinique.

De préférence, on utilise le lauryl sulfate de sodium commercialisé sous la marque TEXAPON K 1296, le disodium cocamido MEA-sulfosuccinate commercialisé sous la marque REWOPOL SBC 212P, et les méthyltaurides d'acides gras, commercialisés par la Société HOECHST.

Les tensio-actifs cationiques conviennent tout particulièrement pour des applications spécifiques telles que le traitement de cheveux sévèrement abîmés et/ou en tant que produits démêlants pour les cheveux.

De préférence, on utilise le produit commercialisé sous la marque N HANCE 3196 par la société HERCULES.

Quant aux tensio-actifs non ioniques, la plupart d'entre eux souffrent de l'inconvénient de n'avoir qu'un faible pouvoir moussant. Il existe cependant des exceptions. Ainsi, on peut citer comme exemples de tensio-actifs non ioniques pouvant être utilisés dans le cadre de la présente invention les polysorbates, et les polyglycérides et polyéthoxylates des alcools gras.

De préférence le produit conforme à la présente invention comprend un tensio-actif non ionique décylpolyglucoside, tel que celui commercialisé sous la marque ORAMIX SP100.

Enfin, les tensio-actifs amphotères peuvent également être utilisés dans le produit selon l'invention, car ils ont un bon pouvoir moussant, et sont de surcroît très doux pour la chevelure. Les dérivés de la bétaïne sont les tensio-actifs amphotères préférés dans le cadre de l'invention. De préférence, on utilise le produit commercialisé sous la marque TEGOBETAÏNE CKD par la Société GOLDSCHMIDT.

Le produit d'hygiène capillaire et/ou corporelle selon l'invention peut également comporter un agent tensio-actif pour augmenter le pouvoir moussant ("foaming agent"). Le monoéthanolamide d'acide gras de coprah, commercialisé sous la marque COMPERLAN 100 est utilisé de préférence.

Comme exemples de sucres pouvant être compris dans le produit selon l'invention, on peut citer le glucose, les sirops de glucose, le mannose, le fructose, le saccharose, le maltose et les sirops de maltose, le lactose, les maltodextrines.

Les amidons susceptibles d'êtres utilisés comme charges dans le produit d'hygiène capillaire et/ou corporelle selon la présente invention comprennent la fécule de pomme de terre et les amidons de blé, de maïs et de riz.

Comme exemples de polyols, on peut citer le sorbitol, le mannitol, le lactitol, le maltitol et notamment les produits commercialisés sous les marques NEOSORB P 60, NEOSORB P 100 T, ou MALTISORB P 90.

A titre d'exemples de protéines et d'acides aminés susceptibles d'être inclus dans le produit d'hygiène capillaire et/ou corporelle selon la présente invention, on peut citer les protéines de lait, dont les caséinates, les protéines végétales telles que les protéines de blé ou de soja, la kératine, notamment celle commercialisée sous la marque MONTEINE WKHP, la cystéine, la méthionine. On peut également adjoindre à la formule des produits tels que la lécithine de soja.

Selon un mode préférentiel de l'invention, la granulométrie de la poudre est comprise entre environ 30 et environ 500 microns, de préférence entre 60 et 300 microns, et plus préférentiellement encore entre 60 et 150 microns.

Le produit d'hygiène capillaire et/ou corporelle en poudre selon la présente invention peut comporter d'environ 0,3 à 12 % d'un parfum, éventuellement encapsulé ou mis sur un support, les pourcentages étant exprimés en poids par rapport au poids total de la poudre. De préférence, la teneur en parfum est comprise entre 1 et 8 %, et plus préférentiellement encore entre 2 et 6 %. Cette possibilité d'inclure dans le produit conforme à l'invention un pourcentage aussi élevé de parfum constitue un avantage déterminant de la présente invention.

Le produit d'hygiène capillaire et/ou corporelle en poudre selon l'invention peut également comporter des vitamines, des colorants, des protéines de traitement, des huiles essentielles encapsulées, selon la spécificité désirée du produit.

Parmi les vitamines, on peut citer les vitamines B2, B5 et B6, la vitamine PP et la vitamine C.

Le produit d'hygiène capillaire et/ou corporelle en poudre selon l'invention peut avantageusement être conditionné sous forme unidose, dans des quantités individuelles de 2 à 10 g, conditionnement qui convient tout particulièrement pour des produits utilisés au cours de voyages, dans des salles de gymnastique, ou offerts dans les hôtels.

Le produit d'hygiène capillaire et/ou corporelle en poudre objet de la présente invention permet d'obtenir une mousse abondante, et permet en plus de procurer un effet exfoliant très agréable pour le lavage du corps.

L'invention a également pour objet un procédé pour la préparation du produit d'hygiène capillaire et/ou corporelle en poudre.

Ce procédé comporte les étapes suivantes :
- on choisit les ingrédients à inclure dans la poudre ;
- le cas échéant, on encapsule ou on met sur support les matières volatiles ou les matières sensibles à la lumière ou à la chaleur telles que parfums, huiles essentielles, vitamines ;
- on réalise un mélange intime des divers ingrédients ;
- on procède éventuellement à un broyage, suivi par un tamisage à la granulométrie choisie ;
- on conditionne le produit.

De façon avantageuse, l'encapsulation ou la mise sur support des matières sensibles se fait sur des maltodextrines, des liposomes, ou des cyclodextrines. On peut citer à titre d'exemple pour les cyclodextrines, les produits commercialisés sous la marque KLEPTOSE par la société ROQUETTE FRERES.

Pour réaliser le mélange on utilise des méthodes connues en soi, telles que l'utilisation d'un mélangeur planétaire.

Le mélange peut être établi à une température entre 5°C et 25°C. De préférence on réalise le mélange à la température ambiante.

Le conditionnement du produit peut se faire, par exemple en sachets ou tubes.

Un grand avantage de ce procédé simple de préparation du produit conforme à l'invention est que l'on peut facilement modifier le contenu de la poudre, l'adaptant ainsi aux besoins spécifiques des consommateurs.

L'invention pourra être mieux comprise grâce à exemple qui suit et qui est donné à titre purement illustratif.

### Exemple :

On prépare un produit d'hygiène capillaire en poudre ayant la composition suivante, en procédant au mélange à sec des différents composants, à une température d'environ 20°C.
- Tensio-actif non ionique en poudré, de marque ORAMIX SP100, commercialisé
   par SEPIC 1,00%
- Tensio-actif anionique en poudre, de marque REWOPOL SBC 212P, commercialisé
   par WITCO 9,00%
- Tensio-actif anionique en poudre, de marque TEXAPON K 1296 commercialisé
   par HENKEL 11,00%
- Produit améliorant du pouvoir moussant, de marque COMPERLAN 100, commercialisé
   par HENKEL 1,00%
- Polymère cationique en poudre de marque
   N HANCE 3196, commercialisé par
   HERCULES 2,20%
- Hydrolysat de fibres de Blé, de marque
   SOFABRAN F 146 (BLE) commercialisé par
   SOFALIA 0,90%
- Kératine, de marque
   MONTEINE W KHP COMMERCIALISE
   PAR SEPPIC 0,20%
- Sorbitol, de marque NEOSORB P60 et
   NEOSORB P100T, commercialisé par ROQUETTE FRERES
   NEOSORB P100T 56,00%
- MALTISORB P90, commercialisé par ROQUETTE FRERES 13,64%
- Parfum 5,00%
- Colorant 0,06%

Le produit est ensuite conditionné dans des tubes de 5 g environ chacun.

10 personnes, 6 femmes et 4 hommes, ont testé le produit. Bien que le pouvoir moussant se manifeste plus lentement qu'avec un shampooing liquide conventionnel, il a été unanimement reconnu comme étant très satisfaisant.

Les expérimentateurs ont apprécié la douceur et la brillance apportées aux cheveux par le shampooing testé, et ont souligné qu'il était extrêmement agréable à utiliser en raison du parfum qui se développe lors de l'emploi et qui subsiste sur la chevelure.

De façon unanime, les expérimentateurs ont également jugé que la présentation sous forme de poudre ne posait aucun problème lors de l'application sur la chevelure et que le produit se révélait tout au contraire très pratique à utiliser.

## Revendications

1. Produit d'hygiène capillaire et/ou corporelle pour l'homme ou l'animal, sous forme de poudre de granulométrie de 30 à 500 µm, **caractérisé par le fait qu'**il est applicable directement sur la chevelure et/ou sur le corps, sous forme de poudre et qu'il comprend, ces pourcentages étant exprimés en poids par rapport au poids total de la poudre, moins de 40%, de préférence moins de 35% et plus préférentiellement encore moins de 30% d'au moins un agent tensio-actif, et 1 à 12 % d'un ou plusieurs parfum(s), le complément à 100% étant constitué par un ou plusieurs produits choisis dans le groupe constitué par les sucres, les amidons, les celluloses, les polyols, les protéines, les acides aminés, les parfums, les colorants, les anti-oxydants, les matières végétales, les algues marines, les vitamines, les huiles essentielles et les charges minérales.

2. Produit d'hygiène capillaire et/ou corporelle selon la revendication 1, **caractérisé par le fait que** le ou les agents tensio-actifs sont choisis dans le groupe constitué par les tensio-actifs anioniques, non ioniques, cationiques, et amphotères.

3. Produit d'hygiène capillaire et/ou corporelle selon l'une ou l'autre des revendications 1 et 2, **caractérisé par le fait que** les tensio-actifs anioniques sont choisis dans le groupe constitué par les alkyl sulfates, les alkyl et aryl sulfonates, les oléfinesulfonates, les alkyl éther sulfates et les esters de l'acide sulfosuccinique, le lauryl sulfate de sodium étant préféré.

4. Produit d'hygiène capillaire et/ou corporelle selon l'une quelconque des revendications 1 à 3, **caractérisé par le fait que** le tensio-actif non ionique est un décylpolyglucoside.

5. Produit d'hygiène capillaire et/ou corporelle selon l'une quelconque des revendications 1 à 4, **caractérisé par le fait que** le tensio-actif amphotère est un dérivé de la betaïne.

6. Produit d'hygiène capillaire et/ou corporelle selon l'une quelconque des revendications 1 à 5, **caractérisé par le fait qu'**il comprend un agent permettant d'augmenter le pouvoir moussant dudit produit, de préférence le monoéthanolamide d'acide gras de coprah.

7. Produit d'hygiène capillaire et/ou corporelle selon l'une quelconque des revendications 1 à 6, **caractérisé par le fait qu'**il comprend un tensioactif cationique ou polymère cationique en tant que produit démêlant pour les cheveux.

8. Produit d'hygiène capillaire et/ou corporelle selon l'une quelconque des revendications 1 à 7, **caractérisé par le fait que** la poudre présente une granulométrie de 30 à 500 microns, de préférence de 60 à 300 microns, et plus préférentiellement encore de 60 à 150 microns.

9. Produit d'hygiène capillaire et/ou corporelle selon l'une quelconque des revendications 1 à 8, **caractérisé par le fait qu'**il comprend au moins un parfum, à une teneur comprise entre 0,3 et 12 %, de préférence entre 1 et 8 %, et plus préférentiellement encore entre 2 et 6 %, les pourcentages étant exprimés en poids par rapport au poids total de la poudre.

10. Produit d'hygiène capillaire et/ou corporelle selon l'une quelconque des revendications 1 à 9, **caractérisé par le fait qu'**il est conditionné sous forme unidose.

11. Procédé de préparation du produit selon l'une quelconque des revendications 1 à 10, **caractérisé par le fait que** :
- l'on choisit les ingrédients à inclure dans la poudre ;
- le cas échéant, l'on encapsule ou l'on met sur support les matières volatiles ou les matières sensibles à la lumière ou à la chaleur, telles que parfums, huiles essentielles, vitamines ;
- l'on réalise un mélange intime des divers ingrédients ;
- l'on procède éventuellement à un broyage suivi par un tamisage à la granulométrie choisie ;
- l'on conditionne le produit.

## Patentansprüche

1. Haar- und/oder Körperpflegemittel für Mensch oder Tier in Puderform mit einer Korngröße von 30 bis 500 µm, **dadurch gekennzeichnet, daß** es in Puderform direkt auf Haare und/oder körper aufbringbar ist und daß es, ausgedrückt in Gewichtsprozent bezüglich des Gesamtgewichts des Puders, weniger als 40 %, vorzugsweise weniger als 35 % und noch mehr bevorzugt weniger als 30 % von wenigstens einem oberflächenaktiven Mittel und 1 bis 12 % von einem oder mehreren Duftstoff(en) umfaßt, wobei der Rest bis 100 % aus einem oder mehreren Produkten besteht, die aus Zuckern, Stärken, Cellulosen, Polyolen, Proteinen, Aminosäuren, Duftstoffen, Farbstoffen, Antioxidantien, Pflanzenstoffen, Meeresalgen, Vitaminen, essentiellen Ölen und mineralischen Füllstoffen ausgewählt sind.

2. Haar- und/oder Körperpflegemittel nach Anspruch 1, **dadurch gekennzeichnet, daß** das oder die oberflächenaktiven Mittel aus anionischen, nichtionischen, kationischen und amphoteren oberflächenaktiven Mitteln ausgewählt sind.

3. Haar- undloder Körperpflegemittel nach einem der Ansprüche 1 oder 2, **dadurch gekennzeichnet, daß** die anionischen oberflächenaktiven Mittel aus Alkylsulfaten, Alkyl- und Arylsulfonaten, Olefinsulfonaten, Alkylethersulfaten und Sulfonbernsteinsäureestern ausgewählt sind, wobei Natriumlaurylsulfat bevorzugt ist.

4. Haar- und/oder Körperpflegemittel nach einem der Ansprüche 1 bis 3, **dadurch gekennzeichnet, daß** das nichtionische oberflächenaktive Mittel ein Decylpolyglucosid ist.

5. Haar- und/oder Körperpflegemittel nach einem der Ansprüche 1 bis 4, **dadurch gekennzeichnet, daß** das amphotere oberflächenaktive Mittel ein Betainderivat ist.

6. Haar- und/oder Körperpflegemittel nach einem der Ansprüche 1 bis 5, **dadurch gekennzeichnet, daß** es ein Mittel umfaßt, durch das sich das Schäumungsvermögen des Produkts steigern läßt, vorzugsweise das Monoethanolamid der Koprafettsäure.

7. Haar- und/oder Körperpflegemittel nach einem der Ansprüche 1 bis 6, **dadurch gekennzeichnet, daß** es ein kationisches oberflächenaktives Mittel oder ein kationisches Polymer als Haar-Entwirrungsmittel umfaßt.

8. Haar- und/oder Körperpflegemittel nach einem der Ansprüche 1 bis 7, **dadurch gekennzeichnet, daß** der Puder eine Korngröße von 30 bis 500 Mikron, vorzugsweise von 60 bis 300 Mikron und noch mehr bevorzugt von 60 bis 150 Mikron aufweist.

9. Haar- und/oder Körperpflegemittel nach einem der Ansprüche 1 bis 8, **dadurch gekennzeichnet, daß** es, ausgedrückt in Gewichtsprozent bezüglich des Gesamtgewichts des Puders, wenigstens einen Duftstoff in einem Gehalt zwischen 0,3 und 12 %, vorzugsweise zwischen 1 und 8 % und noch mehr bevorzugt zwischen 2 und 6 % umfaßt.

10. Haar- und/oder Körperpflegemittel nach einem der Ansprüche 1 bis 9, **dadurch gekennzeichnet, daß** es in einer Dosierungseinheitsform verpackt ist.

11. Verfahren zur Herstellung des Produkts nach einem der Ansprüche 1 bis 10, **dadurch gekennzeichnet, daß**:
- die Bestandteile zum Einbringen in den Puder gewählt werden;
- die flüchtigen Stoffe oder die licht- oder wärmeempfindlichen Stoffe, wie Duftstoffe, essentielle Öle, Vitamine, gegebenenfalls einkapselt oder auf Träger aufgebracht werden;
- eine innige Mischung der verschiedenen Bestandteile hergestellt wird;
- gegebenenfalls eine Zerkleinerung und anschließend eine Siebung bis zur Erzielung der gewählten Korngröße durchgeführt werden;
- das Produkt verpackt wird.

## Claims

1. A hair and/or body care product for humans or animals, in the form of a powder having a granulometry of 30 to 500 microns, **characterized in that** it is directly applicable to the hair and/or the body in the form of powder and comprising, these percentages being expressed as percentages by weight relative to the total weight of the powder, less than 40%, preferably less than 35%, and still more preferably less than 30%, of at least one surfactant, and from 1 to 12% of one or more perfume(s), the percentage being made up to 100% by one or more products selected from the group consisting of sugars, starches, celluloses, polyols, proteins, amino acids, perfumes, colourings, antioxidants, plant substances, seaweed, vitamins, essential oils and mineral fillers.

2. The hair and/or body care product according to claim 1, **characterized in that** the surfactant(s) are selected from the group consisting of anionic, nonionic, cationic and amphoteric surfactants.

3. The hair and/or body care product according to any one of claims 1 or 2, **characterized in that** the anionic surfactants are selected from the group consisting of alkyl sulfates, alkyl and aryl sulfonates, olefin sulfonates, alkyl ether sulfates and the esters of sulfosuccinic acid, sodium lauryl sulfate being preferred.

4. The hair and/or body care product according to any one of claims 1 to 3, **characterized in that** the nonionic surfactant is a decyl polyglucoside.

5. The hair and/or body care product according to any one of claims 1 to 4, **characterized in that** the amphoteric surfactant is a betaine derivative.

6. The hair and/or body care product according to any one of claims 1 to 5, **characterized in that** it comprises an agent increasing the lather-forming capacity of said product, preferably copra fatty acid monoethanolamide.

7. The hair and/or body care product according to any one of claims 1 to 6, **characterized in that** it comprises a cationic surfactant, or a cationic polymer as a hair detangling product.

8. The hair and/or body care product according to any one of claims 1 to 7, **characterized in that** the powder has a granulometry of 30 to 500 microns, preferably 60 to 300 microns, and still more preferably of 60 to 150 microns.

9. The hair and/or body care product according to any one of claim 1 to 8, **characterized in that** comprises at least one perfume, with a content of between 0.3 and 12%, preferably of between 1 and 8%, and still more preferably of between 2 and 6%, the percentages being expressed in weight relative to the total weight of the powder.

10. The hair and/or body care product according to any one of claims 1 to 9, **characterized in that** it is packaged in single dose form.

11. A process for the production of the product according to any one of claims 1 to 10, **characterized in that**:
- the ingredients to be included in the powder are selected;
- where applicable, the volatile ingredients or those sensitive to light or heat, selected from the group consisting of perfumes, essential oils, vitamins, are encapsulated or applied to a support;
- the various ingredients are mixed intimately;
- the mixture is optionally pulverised, and then screened to arrive at the selected granulometry;
- the product is packaged.
